(19) Europäisches Patentamt
European Patent Office
Office européen des brevets

(11) Veröffentlichungsnummer: **0 636 629 A2**

# (12) EUROPÄISCHE PATENTANMELDUNG

(21) Anmeldenummer: **94111097.5**

(22) Anmeldetag: **16.07.94**

(51) Int. Cl.⁶: **C07F 7/22**, C03C 17/25, C23C 16/40

(30) Priorität: **30.07.93 DE 4325648**

(43) Veröffentlichungstag der Anmeldung:
**01.02.95 Patentblatt 95/05**

(84) Benannte Vertragsstaaten:
**AT BE CH DE DK ES FR GB GR IE IT LI LU NL PT SE**

(71) Anmelder: **Th. Goldschmidt AG**
**Goldschmidtstrasse 100**
**D-45127 Essen (DE)**

(72) Erfinder: **Vallerien, Dr. Sven-Uwe**
**Laupendahler Landstrasse 47**
**D-45239 Essen (DE)**

(54) **Alkylzinnverbindungen, deren Herstellung und diese enthaltende Mittel zur Bildung elektrisch leitfähiger und IR-reflektierender Schichten auf Oberflächen von Glas, Glaskeramik oder Email.**

(57) Die Erfindung betrifft Verbindungen der allgemeinen Formel $SnR^1_aR^2_bR^3_c$, in welcher $R^1$ ein Alkylrest mit 1 bis 4 Kohlenstoffatomen, $R^2$ ein Acetatrest, $R^3$ ein Trifluoracetatrest ist, und a einen Wert von 1 oder 2, b einen Wert von 1 oder 2, c einen Wert von 1 oder 2 hat, mit der Maßgabe, daß die Summe a + b + c = 4 ist.

Die Erfindung betrifft ein Verfahren zur Herstellung dieser Verbindungen und ein Mittel zur Herstellung elektrisch leitfähiger und Infrarot-reflektierenderfluordotierter Zinnoxidschichten auf Glas-, Glaskeramik- und/oder Emailoberflächen, wobei es Zinnverbindungen der Formel $SnR^1_aR^2_bR^3_c$ oder Mischungen dieser Zinnverbindungen, wobei die Mischung der allgemeinen Formel $SnR^1_xR^2_yR^3_z$ entspricht, in der x einen Zahlenwert von 1 bis 2, y einen Zahlenwert von 0,1 bis 2,9 und z einen Zahlenwert von 0,1 bis 2,9 hat, und die Summe x + y + z = 4 ist, in einem polaren organischen Lösungsmittel gelöst enthält.

EP 0 636 629 A2

Die Erfindung betrifft neue Alkylzinnverbindungen mit Acetat- und Trifluoracetatgruppen sowie deren Herstellung. Die Erfindung betrifft ferner diese neuen Zinnverbindungen enthaltende Mittel zur Bildung elektrisch leitfähiger und Infrarot-reflektierender Schichten auf Oberflächen von Glas, Glaskeramik oder Email.

Es ist bekannt, daß elektrisch leitfähige, mit Fluor dotierte Zinnoxidschichten auf Glasoberflächen sowohl den elektrischen Widerstand der so beschichteten Oberflächen herabsetzen als auch die Infrarot-Reflexion erhöhen. Zur Erzeugung derartiger Schichten werden geeignete Zinnverbindungen (Basisverbindungen) gleichzeitig mit einer Fluor abgebenden Verbindung (Dotierungsmittel) in Kontakt mit der auf 400 bis 800°C erhitzten Glasoberfläche gebracht. Die Basis-Zinnverbindung bildet eine zusammenhängende Zinnoxidschicht auf der Oberfläche des Glases, der Glaskeramik oder der Emaillierung aus. Das Fluor aus dem Dotierungsmitteli erhöht die elektrische Leitfähigkeit und bewirkt die hohe Infrarotreflexion.

Das Aufsprühen geeigneter Zinn- und fluorhaltiger Lösungen ist zur Aufbringung der mit Fluor dotierten Zinnoxidschichten auf den Oberflächen verfahrenstechnisch besonders einfach.

In der DE-OS 22 46 193 wird ein Verfahren zur Herstellung transparenter, elektrisch leitfähiger Zinnoxidfilme auf Glasoberflächen beschrieben. Dabei wird die Lösung eines Organozinnsalzes der Trifluoressigsäure in Methylethylketon verwendet. Für die Herstellung der Lösungen sind mehrere aufwendige Verfahrensschritte erforderlich. Die nach diesem Verfahren erzeugten Zinncarboxylate weisen einen zu hohen Fluoranteil auf, der jedoch durch die Molekülstruktur vorgegeben ist. Eine Überdotierung mit Fluor führt jedoch zu einer deutlichen Verschlechterung der Infrarotreflexion und der elektrischen Leitfähigkeit.

In der DE-OS 39 15 232 wird ein Verfahren zur Herstellung elektrisch leitfähiger, IR-reflektierender mit Fluor dotierter Zinnoxidschichten durch Aufbringen einer organischen Lösung von Umsetzungsprodukten der Trifluoressigsäure mit Alkylzinnoxiden auf die auf 400 bis 700°C erhitzte Oberfläche beschrieben. Als Lösungsmittel dienen hierbei Essigsäureethylester und/oder Methylethylketon. Die entstehenden Anlagerungsprodukte sind jedoch nicht lagerstabil. Die wünschenswerte Verwendung von polaren niederen Alkoholen, wie z. B. Ethanol oder Isopropanol, als Lösungsmittel, welche, wie dem Fachmann bekannt ist zu optisch fehlstellenfreien, einheitlichen Schichten führen, ist bei diesen Verbindungen nicht möglich.

In der EP-OS 0 318 486 werden Chlorzinnacetattrifluoracetate beschrieben. Diese Verbindungen werden in einem mehrstufigen, aufwendigen Verfahren hergestellt. Die Funktionswerte einer damit erzeugten 200 nm dicken Zinnoxidschicht liegen bei 70 % IR-Reflexion und einem elektrischen Oberflächenwiderstand von 40 Ohm/Quadrat. Hauptnachteil dieser Substanzen ist jedoch die Anwesenheit von Chlor. Bei der Pyrolyse bildet sich u. a. Chlorwasserstoffgas, welches Materialien angreifen oder beschädigen oder Gesundheitsgefährdungen verursachen kann.

Der Erfindung liegt die Aufgabe zugrunde, neuartige Alkylzinnverbindungen aufzufinden, welche in polaren organischen Lösungsmitteln, vorzugsweise niederen aliphatischen Alkoholen oder Ketonen gelöst, ohne zusätzliche Dotierungsmittel den Oberflächenwiderstand der damit auf Glas-, Glaskeramik- und Emailoberflächen erzeugten Zinnoxidschichten minimieren und die IR-Reflexion maximieren. Die Produkte sollen langzeit-lagerstabil sein. Die Produkte sollen frei von Chlor sein.

Überraschenderweise sind diese Eigenschaften bei neuen Zinnverbindungen zu finden, welche erfindungsgemäß der allgemeinen Formel $SnR^1_a R^2_b R^3_c$ entsprechen, in welcher

$R^1$     ein Alkylrest mit 1 bis 4 Kohlenstoffatomen,

$R^2$     ein Acetatrest,

$R^3$     ein Trifluoracetatrest ist, und

a     einen Wert von 1 oder 2,

b     einen Wert von 1 oder 2,

c     einen Wert von 1 oder 2 hat,

mit der Maßgabe, daß die Summe a + b + c = 4 ist.

$R^1$ ist ein Alkylrest mit 1 bis 4 Kohlenstoffatomen, vorzugsweise ein Butylrest.

Beispiele erfindungsgemäßer Verbindungen sind $C_4H_9$-$Sn(CH_3COO)_2 CF_3COO$, $(C_4H_9)_2$-$Sn(CH_3COO)$-$CF_3COO$, $(C_3H_7)$-$Sn(CH_3COO)_2 CF_3COO$.

Bevorzugt sind Verbindungen mit nur einer an Sn gebundenen Alkylgruppe.

Ein weiterer Gegenstand der Erfindung besteht in dem Verfahren zur Herstellung der erfindungsgemäßen Verbindungen, welches dadurch gekennzeichnet ist, daß man in an sich bekannter Weise zu Alkylzinnoxid Essigsäureanhydrid und Trifluoressigsäureanhydrid in den gewünschten molaren Verhältnissen unter Kühlung derart zugibt, daß eine Reaktionstemperatur von 90°C nicht überschritten wird und man nach beendetem Zufügen der Reaktionspartner die Reaktion bei einer Temperatur von etwa 80 bis 90°C während eines Zeitraumes von 2 bis 6 Stunden vervollständigt.

Die Erfindung betrifft ferner ein Mittel zur Herstellung elektrisch leitfähiger und Infrarot-reflektierender fluordotierter Zinnoxidschichten auf Glas-, Glaskeramik- und/oder Emailoberflächen, mit dem Kennzeichen, daß es Zinnverbindungen der Formel $SnR^1_aR^2_bR^3_c$ oder Mischungen dieser Zinnverbindungen, wobei die Mischung der allgemeinen, durchschnittlichen Formel $SnR^1_xR^2_yR^3_z$ entspricht, in der x einen Zahlenwert von 1 bis 2, y einen Zahlenwert von 0,1 bis 2,9 und z einen Zahlenwert von 0,1 bis 2,9, und die Summe x + y + z = 4 ist, in einem polaren organischen Lösungsmittel gelöst enthält.

Vorzugsweise enthält das Mittel Mischungen dieser Zinnverbindungen, in denen x einen Zahlenwert von 1, y einen Zahlenwert von 0,5 bis 2,0 und z einen Zahlenwert von 0,5 bis 2,0, und die Summe x + y + z = 4 ist.

Der Begriff "Mischungen" ist erfindungsgemäß dabei so zu verstehen, daß entsprechend den Molverhältnissen bei der Herstellung der erfindungsgemäßen Verbindungen nicht nur eine einzige Spezies der neuen erfindungsgemäßen Verbindungen, sondern ein Gemisch zweier oder mehrerer der erfindungsgemäßen neuen Verbindungen in dem erfindungsgemäßen Mittel enthalten ist. Die Indices x, y und z geben dann die mittleren Werte der Alkyl-, Acetat- und Trifluoracetat-Gruppen an und können deshalb gebrochene Werte annehmen.

Die erfindungsgemäßen Verbindungen sind in dem erfindungsgemäßen Mittel in polaren organischen Lösungsmitteln gelöst. Das erfindungsgemäße Mittel enthält dabei als polares organisches Lösungsmittel einen aliphatischen Alkohol mit 1 bis 4 Kohlenstoffatomen oder ein Keton mit 2 bis 8 Kohlenstoffatomen. Besonders bevorzugte Lösungsmittel sind Ethanol, Isopropanol und Aceton.

Das erfindungsgemäße Mittel enthält vorzugsweise 40 bis 60 Gew.-% der erfindungsgemäßen Zinnverbindung(en) und 60 bis 40 Gew.-% des organischen Lösungsmittels.

Zur Bildung der gewünschten elektrisch leitfähigen und Infrarot-reflektierenden Schichten wird ein erfindungsgemäßes Mittel in an sich bekannter Weise mittels einer Sprühpistole unter Verwendung von Preßluft im Sprühzerstäubungsverfahren auf die auf 400 bis 800°C erhitzte Glas-, Glaskeramik- oder Emailoberfläche aufgesprüht. Durch Pyrolyse wird auf diesen Oberflächen eine mit Fluor dotierte Zinnoxidschicht erzeugt. Die Dicke dieser Beschichtung kann zwischen 100 nm und 2 $\mu$m variiert werden. Die nach dem erfindungsgemäßen Verfahren hergestellten Schichten zeichnen sich durch eine hohe Transparenz aus. Die integrale Infrarotreflexion im Wellenlängenbereich zwischen 2,5 und 15 $\mu$m liegt über 80 %.

In den nachfolgenden Beispielen werden das Herstell- und das Applikationsverfahren detailliert erläutert.

Beispiel 1

In einem 1-l-Mehrhalskolben, der mit einem KPG-Rührer versehen ist, werden 248,9 g Dibutylzinnoxid eingewogen. Unter langsamem Rühren tropft man 51,1 g Essigsäureanhydrid und 105 g Trifluoressigsäureanhydrid zu (x = 2, y = 1, z = 1). Die Reaktion ist stark exotherm, wobei darauf zu achten ist, daß die Reaktionstemperatur 90°C nicht überschreitet. Nach beendetem Zutropfen wird zur Nachreaktion noch weitere 4 h auf 80 bis 90°C erwärmt.

Das so erhaltene, nahezu klare Produkt wird mit etwas Filterhilfe und Aktivkohle versetzt und über eine AF 2000-Filterplatte mittels Filterpresse filtriert.

Das klare Produkt wird als 50 %ige ethanolische Lösung auf eine Flachglasscheibe (160 mm x 180 mm x 6 mm), die zuvor 5 Minuten bei einer Ofentemperatur von ca. 700°C aufgeheizt wurde, aufgesprüht. Die beschichtete Glasplatte weist bei einer Sprühmenge von 7 ml folgende Werte auf:

| Flächenwiderstand: | 11 Ohm/Quadrat |
|---|---|
| IR-Reflexion: | 87 % |
| Schichtdicke: | 750 nm |

Beispiel 2

In einem 250-ml-Mehrhalskolben, der mit einem KPG-Rührer versetzt ist, werden 149,4 g Dibutylzinnoxid eingewogen. Unter langsamem Rühren tropft man 21,5 g Essigsäureanhydrid und 84,0 g Trifluoressigsäureanhydrid zu (x = 2, y = 1,3, z = 0,7). Die Reaktion ist stark exotherm, wobei darauf zu achten ist, daß die Reaktionstemperatur 90°C nicht überschreitet. Nach beendetem Zutropfen wird zur Nachreaktion noch weitere 4 h auf 80 bis 90°C erwärmt.

Das so erhaltene, nahezu klare Produkt wird mit etwas Filterhilfe und Aktivkohle versetzt und über eine AF 2000-Filterplatte mittels Filterpresse filtriert.

Das klare Produkt wird als 50 %ige ethanolische Lösung auf eine Flachglasscheibe (160 mm x 180 mm x 6 mm), die zuvor 5 Minuten bei einer Ofentemperatur von ca. 700°C aufgeheizt wurde, aufgesprüht.

Die beschichtete Glasplatte weist bei einer Sprühmenge von 7 ml folgende Werte auf:

| Flächenwiderstand: | 26 Ohm/Quadrat |
|---|---|
| IR-Reflexion: | 75 % |
| Schichtdicke: | 1130 nm |

Beispiel 3

In einem 250-ml-Mehrhalskolben, der mit einem KPG-Rührer versetzt ist, werden 149,4 g Dibutylzinnoxid eingewogen. Unter langsamem Rühren tropft man 40,8 g Essigsäureanhydrid und 42,0 g Trifluoressigsäureanhydrid zu (x = 2, y = 0,7, z = 1,3). Die Reaktion ist stark exotherm, wobei darauf zu achten ist, daß die Reaktionstemperatur 90°C nicht überschreitet. Nach beendetem Zutropfen wird zur Nachreaktion noch weitere 4 h auf 80 bis 90°C erwärmt.

Das so erhaltene, nahezu klare Produkt wird mit etwas Filterhilfe und Aktivkohle versetzt und über eine AF 2000-Filterplatte mittels Filterpresse filtriert.

Das klare Produkt wird als 50 %ige ethanolische Lösung auf eine Flachglasscheibe (160 mm x 180 mm x 6 mm), die zuvor 5 Minuten bei einer Ofentemperatur von ca. 700°C aufgeheizt wurde, aufgesprüht.

Die beschichtete Glasplatte weist bei einer Sprühmenge von 10 ml folgende Werte auf:

| Flächenwiderstand: | 53 Ohm/Quadrat |
|---|---|
| IR-Reflexion: | 60 % |
| Schichtdicke: | 1100 nm |

Beispiel 4

In einem 250-ml-Mehrhalskolben, der mit einem KPG-Rührer versetzt ist, werden 104,4 g Monobutylzinnoxid eingewogen. Unter langsamem Rühren tropft man 40,3 g Essigsäureanhydrid und 78,8 g Trifluoressigsäureanhydrid zu (x = 1, y = 1,5, z = 1,5). Die Reaktion ist stark exotherm, wobei darauf zu achten ist, daß die Reaktionstemperatur 90°C nicht überschreitet. Nach beendetem Zutropfen wird zur Nachreaktion noch weitere 4 h auf 80 bis 90°C erwärmt.

Das so erhaltene, nahezu klare Produkt wird mit etwas Filterhilfe und Aktivkohle versetzt und über eine AF 2000-Filterplatte mittels Filterpresse filtriert.

Das klare Produkt wird als 50 %ige ethanolische Lösung auf eine Flachglasscheibe (160 mm x 180 mm x 6 mm), die zuvor 5 Minuten bei einer Ofentemperatur von ca. 700°C aufgeheizt wurde, aufgesprüht.

Die beschichtete Glasplatte weist bei einer Sprühmenge von 7 ml folgende Werte auf:

| Flächenwiderstand: | 79 Ohm/Quadrat |
|---|---|
| IR-Reflexion: | 51 % |
| Schichtdicke: | 840 nm |

Beispiel 5

In einem 250-ml-Mehrhalskolben, der mit einem KPG-Rührer versetzt ist, werden 104,4 g Monobutylzinnoxid eingewogen. Unter langsamem Rühren tropft man 26,9 g Essigsäureanhydrid und 105,0 g Trifluoressigsäureanhydrid zu (x = 1, y = 1, z = 2). Die Reaktion ist stark exotherm, wobei darauf zu achten ist, daß die Reaktionstemperatur 90°C nicht überschreitet. Nach beendetem Zutropfen wird zur Nachreaktion noch weitere 4 h auf 80 bis 90°C erwärmt.

Das so erhaltene, nahezu klare Produkt wird mit etwas Filterhilfe und Aktivkohle versetzt und über eine AF 2000-Filterplatte mittels Filterpresse filtriert.

Das klare Produkt wird als 50 %ige isopropanolische Lösung auf eine Flachglasscheibe (160 mm x 180 mm x 6 mm), die zuvor 5 Minuten bei einer Ofentemperatur von ca. 700°C aufgeheizt wurde, aufgesprüht.

Die beschichtete Glasplatte weist bei einer Sprühmenge von 7 ml folgende Werte auf:

| Flächenwiderstand: | 75 Ohm/Quadrat |
|---|---|
| IR-Reflexion: | 50 % |
| Schichtdicke: | 840 nm |

Beispiel 6

In einem 250-ml-Mehrhalskolben, der mit einem KPG-Rührer versetzt ist, werden 104,4 g Monobutylzinnoxid eingewogen. Unter langsamem Rühren tropft man 53,7 g Essigsäureanhydrid und 52,5 g Trifluoressigsäureanhydrid zu (x = 1, y = 2, z = 1). Die Reaktion ist stark exotherm, wobei darauf zu achten ist, daß die Reaktionstemperatur 90°C nicht überschreitet. Nach beendetem Zutropfen wird zur Nachreaktion noch weitere 4 h auf 80 bis 90°C erwärmt.

Das so erhaltene, nahezu klare Produkt wird mit etwas Filterhilfe und Aktivkohle versetzt und über eine AF 2000-Filterplatte mittels Filterpresse filtriert.

Das klare Produkt wird als 70 %ige Lösung in Aceton auf eine Flachglasscheibe (160 mm x 180 mm x 6 mm), die zuvor 5 Minuten bei einer Ofentemperatur von ca. 700°C aufgeheizt wurde, aufgesprüht.

Die beschichtete Glasplatte weist bei einer Sprühmenge von 5 ml folgende Werte auf:

| Flächenwiderstand: | 87 Ohm/Quadrat |
|---|---|
| IR-Reflexion: | 49 % |
| Schichtdicke: | 1100 nm |

**Patentansprüche**

1. Verbindungen der allgemeinen Formel $SnR^1_aR^2_bR^3_c$, in welcher

   $R^1$ ein Alkylrest mit 1 bis 4 Kohlenstoffatomen,

   $R^2$ ein Acetatrest,

   $R^3$ ein Trifluoracetatrest ist, und

   a einen Wert von 1 oder 2,

   b einen Wert von 1 oder 2,

   c einen Wert von 1 oder 2 hat,

   mit der Maßgabe, daß die Summe a + b + c = 4 ist.

2. Verfahren zur Herstellung der Verbindungen des Anspruchs 1 oder ihrer Mischungen, dadurch gekennzeichnet, daß man in an sich bekannter Weise zu Alkylzinnoxid Essigsäureanhydrid und Trifluoressigsäureanhydrid in den gewünschten molaren Verhältnissen unter Kühlung derart zugibt, daß eine Reaktionstemperatur von 90°C nicht überschritten wird und man nach beendetem Zufügen der Reaktionspartner die Reaktion bei einer Temperatur von etwa 80 bis 90°C während eines Zeitraumes von 2 bis 6 Stunden vervollständigt.

3. Mittel zur Herstellung elektrisch leitfähiger und Infrarot-reflektierender fluordotierter Zinnoxidschichten auf Glas-, Glaskeramik- und/oder Emailoberflächen, dadurch gekennzeichnet, daß es Zinnverbindungen der Formel $SnR^1_aR^2_bR^3_c$ oder Mischungen dieser Zinnverbindungen, wobei die Mischung der allgemeinen Formel $SnR^1_xR^2_yR^3_z$ entspricht, in der x einen Zahlenwert von 1 bis 2, y einen Zahlenwert von 0,1 bis 2,9 und z einen Zahlenwert von 0,1 bis 2,9, und die Summe x + y + z = 4 ist, in einem polaren organischen Lösungsmittel gelöst enthält.

4. Mittel nach Anspruch 3, dadurch gekennzeichnet, daß es Mischungen dieser Zinnverbindungen, in denen x einen Zahlenwert von 1, y einen Zahlenwert von 0,5 bis 2,0 und z einen Zahlenwert von 0,5 bis 2,0, und die Summe x + y + z = 4 ist, enthält.

5. Mittel nach Anspruch 3 oder 4, dadurch gekennzeichnet, daß es als polares organisches Lösungsmittel einen aliphatischen Alkohol mit 1 bis 4 Kohlenstoffatomen oder ein Keton mit 2 bis 8 Kohlenstoffatomen enthält.

6. Mittel nach Anspruch 3, 4 oder 5, dadurch gekennzeichnet, daß es
40 bis 60 Gew.-% der Zinnverbindung oder des Gemisches der Zinnverbindungen und
60 bis 40 Gew.-% des organischen Lösungsmittels enthält.